(19) Europäisches Patentamt
European Patent Office
Office européen des brevets

(11) **EP 3 659 547 B1**

(12) **EUROPÄISCHE PATENTSCHRIFT**

(45) Veröffentlichungstag und Bekanntmachung des Hinweises auf die Patenterteilung:
**17.11.2021  Patentblatt 2021/46**

(51) Int Cl.:
*A61C 13/00* $^{(2006.01)}$  *A61C 13/08* $^{(2006.01)}$
*B28B 1/00* $^{(2006.01)}$  *B29C 64/00* $^{(2017.01)}$

(21) Anmeldenummer: **18209271.8**

(22) Anmeldetag: **29.11.2018**

(54) **VERFAHREN UND VERWENDUNG VON SCHLICKER ZUR HERSTELLUNG VON KERAMIKFORMKÖRPERN AUS ZIRKONOXID DURCH 3D-INKJET-DRUCKEN**

METHOD AND USE OF SLIP FOR PRODUCING CERAMIC MOULDED BODIES MADE OF ZIRCONIUM BY MEANS OF 3D INK JET PRINTING

PROCÉDÉ ET UTILISATION DE BARBOTINE DESTINÉS À LA FABRICATION DE CORPS MOULÉS CÉRAMIQUES À PARTIR DE L'OXYDE DE ZIRCONIUM PAR IMPRESSION TRIDIMENSIONNELLE À JET D'ENCRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.06.2020   Patentblatt 2020/23**

(73) Patentinhaber: **Ivoclar Vivadent AG**
**9494 Schaan (LI)**

(72) Erfinder:
• **RITZBERGER, Christian**
**9472 Grabs (CH)**

• **RENNER, Johannes**
**3072 Ostermundingen (CH)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 2 157 067      WO-A1-2018/040834**
**DE-A1-102015 110 360    US-A1- 2004 075 197**
**US-A1- 2013 313 738    US-A1- 2014 109 797**
**US-A1- 2018 170 812**

**Beschreibung**

Die vorliegende Erfindung betrifft ein Verfahren und Verwendung eines Schlickers zur Herstellung von Keramikformteilen aus Zirkonoxidkeramik, insbesondere von dentalen Restaurationen, durch ein 3D-Inkjet-Druckverfahren.

Zur Herstellung von Formkörpern werden in zunehmendem Maße sogenannte aufbauende Verfahren eingesetzt. Als Synonym für diese aufbauenden Verfahren werden oftmals die Begriffe "rapid prototyping", "generative Fertigungsverfahren" oder "additive Fertigungsverfahren" verwendet. Unter diesen Begriffen werden verschiedene Verfahren zusammengefasst, bei denen aus computergestützten Konstruktionsdaten (CAD-Daten) 3-dimensionale Modelle oder Bauteile hergestellt werden (A. Gebhardt, Vision Rapid Prototyping, Ber. DGK 83 (2006) 7-12). Beispiele für typische additive Fertigungsverfahren sind die Stereolithographie, das 3D-Printing und das Inkjet-Modeling. Das Prinzip des Rapid Prototyping beruht auf dem schichtweisen Aufbau eines dreidimensionalen Bauteils.

Unter den 3D-Tintenstrahldruckverfahren ist das Drucken polymerisierbarer Tinten eines von mehreren Verfahren. Daneben sind auch das Drucken eines Binders in ein Pulverbett oder das Drucken flüssiger Wachse bekannt. Bei den 3D-Tintenstrahldruckverfahren werden, demselben Prinzip wie die aus dem Büroalltag bekannten klassischen Tintenstrahldrucker folgend, direkt 3D-Objekte gedruckt, indem durch einen Piezo-Druckkopf, der mehrere Düsen aufweisen kann, polymerisierbare Modellmaterialien ("Tinten") in definierten Tropfen abgegeben werden, die Tinte ausgehärtet und so eine Schicht mit der gewünschten gedruckten Kontur gebildet wird.

Beim Tintenstrahldrucken können Piezo-Druckköpfe eingesetzt werden, die einen Tintenzulauf und eine Mehrzahl von mit dem Tintenzulauf verbundenen Düsen aufweisen, wobei jeder Düse ein Piezoelement zugeordnet ist. Die Piezoelemente der einzelnen Düsen sind von einer Steuereinheit separat ansteuerbar. Durch Beaufschlagung der Piezoelemente mit Steuersignalen werden die Piezoelemente gezielt verformt, um durch die Verformung einen diskreten Tropfen der Tinte durch die Düse mit definierter Tropfengröße auszustoßen. Größe und Volumen der Tropfen sowie die Tropfenabfolge aus der Düse lassen sich dabei durch die angelegten elektrischen Impulse steuern. Die Arbeitsfrequenz eines Piezoelements reicht aufgrund der Geometrie des Druckkopfes und der Rheologie der Tinten in der Regel bis etwa 20 kHz. Die Summe der Tropfen auf einem Substrat ergibt die gewünschte definierte zweidimensionale Struktur.

Die Düsen eines solchen Druckkopfes haben typischerweise eine sehr kleine Öffnung, die im Größenbereich von 10 bis 100 $\mu$m liegt, weshalb die ausgestoßenen Tropfen konsequenterweise sehr klein sind; ihr Durchmesser entspricht in erster Näherung der Öffnung der Düse, ihr Volumen liegt im Pikoliter-Bereich ($10^{-10}$ bis $10^{-12}$ Liter). Daher ist es möglich, in solchen Druckprozessen sehr feine Strukturen mit hoher Auflösung zu drucken. Im Fall des 3D-Tintenstrahldrucks werden dazu üblicherweise dünnflüssige, polymerisierbare, meist photopolymerisierbare Substanzen als Tinten eingesetzt, die unmittelbar nach Auftreffen der Tropfen auf dem Substrat ausgehärtet werden. Typischerweise geschieht das, indem ein oder mehrere Druckköpfe über ein Substrat hinwegbewegt werden, die eine flächige Struktur bestehend aus einer Vielzahl aneinander gereihter Tropfen einer mit Photoinitiatoren versehenen polymerisierbaren Tinte drucken, und nachfolgend (oder parallel dazu) eine Belichtung mit einer Lichtquelle durchgeführt wird, die Licht mit einer geeigneten Wellenlänge emittiert, um das photopolymerisierbare Material der Tinte zu polymerisieren.

Tinten für solche 3D-Tintenstrahldruckverfahren enthalten in der Regel Füllstoffe. Füllstoffe können zum Beispiel Farbpigmente, Trübungsmittel etc. sein, die die optischen Eigenschaften der Tinte verändern. Durch Füllstoffe können aber auch die Fließeigenschaften der Tinte beeinflusst oder die Rheologie gesteuert werden. Im Fall des 3D-Tintenstrahldruckens zum Aufbau von keramischen Formkörpers sind Füllstoffpartikel notwendig. Die Füllstoffpartikel sind in diesem Fall das eigentliche Baumaterial (z.B. Oxidkeramik). Die sie umgebende Flüssigkeit ist lediglich die Matrix, um diese Partikel mit der beschriebenen Technik zu einem Formkörper aufzubauen. Die Partikel beeinflussen dabei die Aspektverhältnisse (Dicken bzw. Schichthöhen) nicht direkt, jedoch die mechanischen, optischen, thermischen und elektrischen Eigenschaften des Formkörpers.

Möchte man 3D-Formkörper aus einem bestimmten Material, wie zum Beispiel Metall oder Keramik, erzeugen, so sind Füllstoffpartikel nicht nur Hilfsstoffe zum Einstellen bestimmter Dimensionen oder mechanischen Eigenschaften des Formkörpers, sondern essentieller Bestandteil der Tinte, und die flüssigen Bestandteile der Tinte fungieren als eine Art Trägersubstanz. Dabei sind in erster Näherung die Dimensionen und mechanischen Eigenschaften des dreidimensionalen Formkörpers umso besser erreichbar, je höher der Feststoffanteil in der Suspension ist, also je mehr Partikel als Füllstoffe in der Tinte enthalten sind. Typischerweise liegen die Teilchengrößen im Bereich von 0,1 bis 1 $\mu$m, die sich insbesondere zum Erreichen eines hohen Füllgrades eignen. Ein hoher Anteil an Feststoffpartikeln kann sich jedoch auf zweierlei Weise negativ auf die Tinte bzw. in der Folge auf den Tropfenausstoß/Druckprozess auswirken. Zum einen nimmt die Viskosität der Tinte mit zunehmendem Füllgrad zu und damit verschlechtern sich die Fließeigenschaften der Tinte. Zum anderen besteht die Gefahr, dass die feinen Düsen verstopft oder teilweise zugesetzt werden, wodurch der Tropfenausstoß verhindert oder behindert werden kann.

Keramikgefüllte Tinten, sogenannte Schlicker, die sich zum Aufbau von Dentalrestaurationen eignen und die im Zusammenhang mit der vorliegenden Erfindung in 3D-Tintenstrahldruckverfahren verwendbar sind, sind in EP 2 233 449 A1 beschrieben. Diese Tinten können Keramikpartikel, Wachs und mindestens ein radikalisch polymerisierbares Monomer enthalten. Zu weiteren Einzelheiten dieser Materialien wird auf die genannte veröffentlichte Patentanmeldung verwiesen.

Aus der EP 2 529 694 A1 ist ein Verfahren zur Herstellung von Keramikformteilen durch dreidimensionales Drucken bekannt, bei dem mindestens zwei unterschiedlich zusammengesetzte Keramikschlicker verwendet werden. Das Aufbringen der Keramikschlicker erfolgt so, dass die relativen Anteile der Schlicker ortsabhängig so gesteuert werden, dass sie innerhalb einer Schicht entlang wenigstens einer Richtung in der Schichtebene in vorgebbarer Weise variieren, wobei das Aufbringungsschema der Schlicker von Schicht zu Schicht variieren kann. Auf diese Weise lassen sich Formkörper herstellen, deren Materialeigenschaften in drei Raumrichtungen variieren.

Die WO 2015/056230 A1 offenbart ein Verfahren zur Herstellung dreidimensionaler Objekte durch das Verdrucken eines Schlickers, der eine Trägerflüssigkeit und ein Dispergiermittel enthält. Die Temperatur der zuletzt gedruckten Schicht wird so eingestellt, dass sie über dem Siedepunkt der Trägerflüssigkeit und unter dem Siedepunkt des Dispergiermittels liegt. Auf diese Weise wird die Trägerflüssigkeit bereits während des Druckvorgangs verdampft, während zumindest ein Teil des Dispergiermittels in der gedruckten Schicht verbleibt und die Partikel des Schlickers zusammenhält.

Die durch schichtweisen Aufbau erhaltenen, ungesinterten Körper werden als Grünkörper oder Grünling bezeichnet. Ein Nachteil der bekannten Verfahren ist, dass diese Grünkörper nach dem Druckvorgang in einem zusätzlichen Schritt entbindert werden müssen, bevor sie abschliessend gesintert werden können. Beim Entbindern werden die organischen Bestandteile der Tinten durch thermische Zersetzung weitgehend entfernt. Durch die Zersetzung der organischen Bestandteile werden die Formkörper hohen Spannungen ausgesetzt, die zur Bildung von Mikrorissen führen können, welche die Gebrauchseigenschaften der Körper beeinträchtigen oder diese gar ganz unbrauchbar machen. Die Aufgabe der Erfindung besteht darin, die Nachteile des Standes der Technik zu überwinden und Schlicker und Verfahren zur Herstellung von Keramikkörpern aus Zirkonoxidkeramik durch 3D-Druck zur Verfügung zu stellen, die einen separaten Entbinderungsschritt überflüssig machen.

Die Aufgabe wird durch die Verwendung von Schlickern gelöst, die Zirkonoxid mit einer Teilchengröße von 50 bis 250 nm enthalten, das in einem flüssigen Medium suspendiert ist. Der Schlicker weist einen Gehalt an Zirkonoxid von 68 bis 88 Gew.-%, bevorzugt 70 bis 86 Gew.-% und besonders bevorzugt 75 bis 85 Gew.-% auf. Der Schlicker ist dadurch gekennzeichnet, dass er nicht mehr als 3 Gew.-%, bevorzugt nicht mehr als 2 Gew.-% und besonders bevorzugt nicht mehr als 1 Gew.-% an organischen Komponenten enthält, bezogen auf die Menge an Feststoff in dem Schlicker. Der Schlicker hat eine Viskosität von 5 bis 1000 mPas und wird im Folgenden auch als Suspension bezeichnet.

Die erfindungsgemäße Aufgabe wird weiterhin durch ein Verfahren zur Herstellung von keramischen Bauteilen gelöst, bei dem man

(i) den Schlicker schichtweise zur geometrischen Form gewünschten Bauteils formt, wobei man vorzugsweise die einzelnen Schichten nach dem Drucken jeweils trocknet,
(ii) man den so erhaltenen Grünling ggf. anschließend trocknet und
(iii) man dann den Grünling ggf. sintert.

Der Aufbau des Grünlings in Schritt (i) erfolgt vorzugsweise durch ein schichtweises Inkjet-Druckverfahren. Bei der Dimensionierung des Bauteils ist der spätere Sinterschrumpf zu berücksichtigen. Gemäß einer bevorzugten Ausführungsform des Verfahrens werden die einzelnen Schichten nach dem Verdrucken jeweils getrocknet. Das Trocknen der Schichten erfolgt vorzugsweise mit einem Luftstrom und/oder durch IR-Strahlung.

In Schritt (ii) wird der in Schritt (i) erhaltene Grünling vorzugsweise (weiter) getrocknet, wobei nach dem Trocknen eine gewisse Restfeuchte verbleiben kann. Ziel des Trocknens ist es, den zuvor flüssigen Schlicker zu verfestigen.

Insbesondere betrifft die Erfindung ein Verfahren zum schichtweisen Aufbau eines Formkörpers mittels eines 3D-Tintenstrahldruckverfahrens mit einem Piezo-Druckkopf, der einen Tintenzulauf und mehrere damit verbundene Düsen aufweist, denen jeweils ein Piezo-Element zugeordnet ist, das auf die zugehörige Düse einwirken kann, um Tinte daraus auszustoßen, wobei jedes Piezo-Element des Piezo-Druckkopfes von einer Steuereinheit individuell zum Ausstoß von Tinte ansteuerbar ist und ihr Ausstoß von der Steuereinheit überwacht wird, wobei bei dem Verfahren der Piezo-Druckkopf gesteuert von der Steuereinheit über ein Baugebiet verfahren wird, die Piezo-Elemente dabei von der Steuereinheit individuell zur ortselektiven Aufbringung von gefüllter Tinte angesteuert werden, um so eine Schicht mit von der Steuereinheit vorgegebener Kontur aufzubringen, die aufgebrachte Schicht aushärten gelassen wird und durch aufeinanderfolgende Aufbringung von Schichten mit jeweils vorgegebener Kontur der gewünschte Formkörper aufgebaut wird.

Vorzugsweise ist die Steuereinheit dazu eingerichtet, nach einer vorgegeben Anzahl von gedruckten Schichten und/oder falls die Steuereinheit bei einer Düse des Piezo-Druckkopfs einen verminderten Ausstoß von Tinte erfasst, den Piezo-Druckkopf aus dem Baugebiet herauszufahren, anschließend die Düse oder Düsen mit vermindertem Ausstoß oder alle Düsen des Piezo-Druckkopfs einer Düsenreinigung zu unterziehen, indem das oder die Piezo-Elemente der zu reinigenden Düsen mit einer Frequenz von wenigstens 20 kHz zur Erzeugung von Ultraschallwellen angeregt werden, um eventuelle Ablagerungen in der Düse zu zerkleinern und zu lösen.

Der Schlicker und das erfindungsgemäße Verfahren eignen sich zur Herstellung keramischer Bauteile aus Zirkonoxidkeramik, insbesondere zur Herstellung von Dentalrestaurationen, wie z.B. einem Inlay, Onlay, Veneer, einer Krone, Brücke, einem Gerüst, Implantat, einer Schale oder einem Abutment.

Der Schlicker und das Verfahren eignen sich aber auch zur Herstellung anderer medizinischer Prothesen, wie Hüft- oder Knieprothesen und von orthopädischem Knochenersatz.

Außerdem eignen sich der Schlicker und das Verfahren zur Herstellung komplexer keramischer Bauteile für den Maschinenbau.

Das **Zirkonoxid** in der Suspension liegt in partikulärer Form vor und hat eine Teilchengröße von 50 bis 250 nm, bevorzugt von 60 bis 250 nm und besonders bevorzugt 80 bis 250 nm, gemessen als $d_{50}$-Wert, bezogen auf das Volumen aller Teilchen. Die Bestimmung der Teilchengröße erfolgt insbesondere mit dem Verfahren der statischen Laserbeugung (SLS) nach ISO 13320:2009, z.B. unter Benutzung eines Partikelanalysators LA-960 der Firma Horiba, oder der dynamischen Lichtstreuung (DLS) nach ISO 22412:2017, z.B. unter Benutzung eines Partikelmessgeräts Nano-flex der Firma Colloid Metrix. $d_{50}$ bedeutet, dass 50% der Partikel kleiner und 50% der Partikel größer als der angegebene Wert sind.

Die Keramikpartikel sollten deutlich kleiner als der mittlere Durchmesser der Düse des Druckkopfs des verwendeten Inkjet-Druckers sein, mit dem der Schlicker verdruckt wird. Um das Verdrucken mit gängigen Inkjet-Druckern, die einen Düsendurchmesser von etwa 100 $\mu$m oder kleiner aufweisen, zu ermöglichen, werden in dem erfindungsgemäßen Schlicker bevorzugt Keramikpartikel mit einer maximalen Partikelgröße von kleiner oder gleich 5 $\mu$m, insbesondere kleiner oder gleich 1 $\mu$m eingesetzt. Vorzugsweise weisen die Partikel eine Größe von 0,01 bis 5 $\mu$m, besonders bevorzugt von 0,01 bis 2 $\mu$m und ganz besonders bevorzugt von 0,01 bis 1, $\mu$m auf, wobei hier die absoluten Ober- und Untergrenzen der Größe der Keramikteilchen gemeint sind.

Die Primärpartikelgröße des Zirkonoxids liegt insbesondere im Bereich von 30 bis 100 nm und sie wird üblicher Weise ebenfalls mit einem wie vorstehend beschriebenen Verfahren der dynamischen Lichtstreuung (DLS) oder mittels Rasterelektronenmikroskopie bestimmt.

Bei dem Zirkonoxid handelt es sich insbesondere um Zirkonoxid auf Basis von polykristallinem tetragonalem Zirkonoxid (TZP). Bevorzugt ist Zirkonoxid, das mit $Y_2O_3$, $La_2O_3$, $CeO_2$, MgO und/oder CaO stabilisiert ist und insbesondere mit 2 bis 14 Mol-%, vorzugsweise mit 2 bis 10 Mol-% und besonders bevorzugt 2 bis 8 Mol.-% und ganz besonders bevorzugt 3 bis 6 Mol.-% dieser Oxide, bezogen auf den Gehalt an Zirkonoxid, stabilisiert ist.

Das im erfindungsgemäßen Verfahren eingesetzte Zirkonoxid kann auch eingefärbt sein. Die gewünschte Einfärbung wird dabei insbesondere erzielt, indem das Zirkonoxid mit einem oder mehreren färbenden Elementen versetzt wird. Die Zugabe von färbenden Elementen wird bisweilen auch als Dotierung bezeichnet und sie erfolgt üblicherweise während der Herstellung des Zirkonoxidpulvers durch Cofällung und anschließende Kalzinierung. Beispiele für geeignete färbende Elemente sind Fe, Mn, Cr, Ni, Co, Pr, Ce, Eu, Gd, Nd, Yb, Tb, Er und Bi.

Bei dem Zirkonoxid in der Suspension kann es sich auch um ein Gemisch von Zirkonoxidpulvern mit unterschiedlicher Zusammensetzung handeln, die insbesondere zu einer unterschiedlichen Färbung und/oder Transluzenz bei der schließlich erzeugten dentalen Restauration führt. Mithilfe eines Gemisches aus unterschiedlich gefärbten Zirkonoxidpulvern kann somit die gewünschte Farbe für den gewünschten Formkörper einfach und in gezielter Weise eingestellt werden. In gleicher Weise kann auch die Transluzenz der erzeugten Formkörper durch Verwendung eines Gemisches von Zirkonoxidpulvern unterschiedlicher Transluzenz gezielt eingestellt werden. Der Grad der Transluzenz der erzeugten Formkörper kann insbesondere durch den Gehalt an Yttriumoxid der verwendeten Zirkonoxidpulver gesteuert werden.

Bei der Suspension kann es sich auch um ein Gemisch von unterschiedlichen Suspensionen zum Beispiel mit unterschiedlich gefärbtem Zirkonoxid handeln, wobei die unterschiedlichen Suspensionen vor dem Verdrucken oder während des Druckprozesses miteinander gemischt werden können. Ein Mischen während des Druckvorgangs kann beispielsweise dadurch erfolgen, dass zwei oder mehrere unterschiedliche Suspensionen gleichzeitig aber jeweils mit separaten Druckköpfen verdruckt werden.

Im erfindungsgemäßen Verfahren liegt das Zirkonoxid als Suspension in einem **flüssigen Medium** vor. Das flüssige Medium kann organische und/oder anorganische Lösungsmittel enthalten. Ein bevorzugtes anorganisches Lösungsmittel ist Wasser. Bevorzugte organische Lösungsmittel sind mit Wasser mischbare Lösungsmittel, insbesondere Alkohole, Ketone, Ester, Ether und Mischungen davon. Mischungen von Wasser mit einem oder mehreren organischen Lösungsmitteln können ebenfalls eingesetzt werden. Das flüssige Medium enthält insbesondere Wasser, wobei solche Suspensionen ganz besonders bevorzugt sind, die als Lösungsmittel ausschließlich Wasser enthalten.

Die Schlicker zeichnen sich dadurch aus, dass der Schlicker lediglich geringe Mengen an organischen Komponenten aufweist, d.h. es enthält organische Komponenten in einer Menge von nicht mehr als 3 Gew.-%, bevorzugt nicht mehr als 2 Gew.-% und besonders bevorzugt nicht mehr als 1 Gew.-%, bezogen auf die Menge an Feststoff in der Suspension.

In einer weiteren bevorzugten Ausführungsform enthält das flüssige Medium organische Komponenten in einer Menge von 0,05 bis 3 Gew.-%, insbesondere 0,1 bis 3 Gew.-%, besonders bevorzugt 0,1 bis 2 Gew.-% und besonders bevorzugt 0,1 bis 1 Gew.-%, bezogen auf die Menge an Feststoff in der Suspension.

Als organische Komponenten kommen insbesondere Dispergiermittel, Bindemittel, Mittel zur Einstellung des pH-Wertes, Stabilisierungsmittel und/oder Entschäumer infrage.

Das **Dispergiermittel** dient dazu, die Agglomeration von suspendierten Teilchen zu größeren Teilchen zu vermeiden. Die Menge an Dispergiermittel in dem Schlicker beträgt insbesondere 0,01 bis 5 Gew.-%, bevorzugt 0,1 bis 2 Gew.-% und besonders bevorzugt 0,1 bis 1 % Gew.-%, bezogen auf die Menge an Feststoff in der Suspension.

Geeignete Dispergiermittel sind wasserlösliche Polymere, wie Polyvinylalkohole, Polyethylenimine, Polyacrylamide, Polyethylenoxide, Polyethylenglykole, Homo- und Copolymere der (Meth)acrylsäure, Polyvinylpyrrolidon, Biopolymere, wie Stärke, Alginate, Gelatine, Celluloseether, wie Carboxymethylcellulose, Vinylsulfonsäure- und Vinylphosphonsäure.

Bevorzugte Dispergiermittel sind Aminoalkohole, wie Ethanolamin, Glykole, wie Ethylenglykol und Dipropylenglykol, Carbonsäuren, wie Maleinsäure und Citronensäure, und Carbonsäuresalze, sowie Mischungen dieser Dispergiermittel.

Es ist weiter bevorzugt, dass der Schlicker als Dispergiermittel mindestens eine Verbindung ausgewählt aus Aminoalkohol, Glykol, Carbonsäure und Carbonsäuresalz enthält. Besonders bevorzugt enthält der Schlicker mindestens eine Verbindung ausgewählt aus Ethanolamin, Ethylenglykol, Dipropylenglykol, Citronensäure und Citronensäuresalz.

Besonders bevorzugte Dispergiermittel sind Polycarbonsäuren, Carbonsäuren, Polycarboxylate, Carboxylate und/oder Amine. Ganz besonders bevorzugt sind Zitronen-, Essig-, Maleinsäure, Ammoniumcitrat, Diammoniumcitrat, Triammoniumcitrat, Ammoniummaleat, Diammoniummaleat und Ammoniumformiat.

Ganz besonders bevorzugte Amine sind Ethanolamin, Diethanolamin und Triethanolamin.

Das **Bindemittel** fördert den Zusammenhalt von Teilchen in dem nach Schritt (i) vorliegenden Grünkörper. Die Menge an Bindemittel in dem Schlicker beträgt insbesondere 0,01 bis 5 Gew.-%, bevorzugt 0,01 bis 3 Gew.- und besonders bevorzugt 0,01 bis 2 Gew.-%, bezogen auf die Menge an Feststoff in der Suspension.

Beispiele für geeignete Bindemittel sind Methylcellulose, Natriumcarboxymethylcellulose, Stärken, Dextrine, Natriumalginat, Ammoniumalginat, Polyethylenglykole, Polyvinylbutyral, Acrylatpolymere, Polyethylenimin, Polyvinylalkohol, Polyvinylpyrrolidon und Mischungen davon.

Bevorzugte Bindemittel sind Polyvinylalkohol, Polyvinylacetat, Polyvinylpyrrolidon, Polyacrylsäure, Copolymere von Acrylsäureester und Acrylsäure, Polyethylacrylat, Polymethacrylsäure, Polymethylmethacrylat, Ammoniumpolyacrylat, Ammoniumpolymethacrylat, Polyethylenglykol und feste Copolymere aus Ethylenglykol und Propylenglykol.

Besonders bevorzugte Bindemittel sind Polyglykole und Glykole, ganz besonders bevorzugt Dipropylenglykol und/oder Polyethylenglykol.

Es ist ein weiterer Vorteil des durch das erfindungsgemäße Verfahren erhältlichen Grünlings, dass er trotz geringer Anteile an Bindemittel auch ohne vorherige Vorsinterung über eine für die Weiterverarbeitung ausreichende Festigkeit verfügt.

Als **Mittel zur Einstellung des pH-Werts** und als **Stabilisierungsmittel** kommen insbesondere Säuren und Basen infrage, wie Carbonsäuren, z.B. 2-(2-Methoxyethoxy)essigsäure und 2-[2-(2-Methoxyethoxy)ethoxy]essigsäure, anorganische Säuren, z.B. Salzsäure und Salpetersäure, sowie Ammoniumhydroxid und Tetramethylammoniumhydroxid. Es ist bevorzugt, dass das flüssige Medium Tetramethylammoniumhydroxid enthält.

Bevorzugte Mittel zur Einstellung des pH-Wertes sind Säuren, Basen und deren Salze. Ganz besonders bevorzugt sind $HNO_3$, HCl, $NH_4OH$, 2,2-Methoxyethoxycitronenäsure, 2,2,2-Methoxyethoxyethoxycitronenäsure und/oder Tetramethylammoniumhydroxid.

Der **Entschäumer** dient der Vermeidung von Luftblasen in der Suspension. Er wird typischerweise in einer Menge von 0,00001 bis 1 Gew.-%, vorzugsweise 0,00001 bis 0,5 Gew.-% und besonders bevorzugt 0,001 bis 0,1 Gew.-%, bezogen auf die Menge an Feststoff in der Suspension, in dem flüssigen Medium verwendet. Beispiele für geeignete Entschäumer sind Paraffin, Siliconöle, Alkylpolysiloxane, höhere Alkohole, Propylenglykol, Ethylenoxid-Propylenoxid-Addukte und insbesondere Alkylpolyalkylenglykolether.

Die Suspension weist eine Viskosität von 5 mPas bis 500 mPas, bevorzugt 5 mPas bis 250 mPas und besonders bevorzugt 5 bis 100 mPas auf. Die Viskosität wird, wenn nicht anders angegeben, mit einem Rotationsviskosimeter mit Platten-Kegel-System, Durchmesser 50 mm und Winkel 1° (MCR302-Modular Compact Rheometer, Firma Anton Paar GmbH), bei einer Scherrate im Bereich von 0,1-1000 $s^{-1}$ und einer Temperatur von 25°C gemessen.

Zur Erzeugung der Suspension wird typischerweise das Zirkonoxid in Pulverform mit dem flüssigen Medium innig vermischt. Dabei können auch Gemische von zum Beispiel unterschiedlich gefärbtem Zirkonoxid verwendet werden. Bei dieser Vermischung werden üblicherweise auch vorhandene Agglomerate aufgebrochen und es kann auch ein Mahlen des eingesetzten Zirkonoxids erfolgen, um die gewünschte Teilchengröße zu erzeugen. Das Vermischen von Zirkonoxid und flüssigem Medium kann daher zum Beispiel vorteilhaft in Rührwerkskugelmühlen durchgeführt werden.

Es können jedoch auch Agglomerate von Keramikprimärteilchen im Schlicker vorliegen, solange sie genügend klein sind, um mit den gewünschten Inkjet-Düsen verdruckt werden zu können, d.h. in bevorzugten Ausführungsformen erfüllen die Agglomerate als Ganzes die obigen Partikelgrößenbedingungen. Es ist jedoch bevorzugt, dass die Partikel in nicht agglomerierter Form vorliegen, beispielsweise ganz oder überwiegend in Form von Primärpartikeln.

Die erfindungsgemäßen Schlicker enthalten vorzugsweise:

| [Gew.-%] | Bestandteil |
|---|---|
| 15 - 30 | Wasser |
| 70 - 85 | $ZrO_2$-Partikel |
| 0,01 - 0,5 | Polycarbonsäure, Carbonsäure, Polycarboxylat und/oder Carboxylat (Dispergiermittel) |
| 0,001 -0,5 | Amin (Dispergiermittel) |
| 0,1 - 2,0 | Polyglykol und/oder Glykol (Bindemittel) |
| 0,01 - 1,0 | Säure, Base oder ein Salz davon (Mittel zur Einstellung des pH-Wertes) |

In allen Fällen sind solche Schlicker bevorzugt, die die oben definierten bevorzugten und besonders bevorzugten Bestandteile enthalten.

Die Schlicker sind besonders zur Verwendung beim Inkjet-Drucken geeignet, grundsätzlich können sie aber auch in anderen Verfahren eingesetzt werden, etwa kann ein Grünkörper aus dem erfindungsgemäßen Schlicker auch nach einem anderen generativen Fertigungsverfahren, wie etwa durch Stereolithographie, oder im Heißgießverfahren (Niederdruckspritzguss) oder durch ein Drop-on-Powder-Verfahren (Drucken in ein Pulverbett) hergestellt werden. Bei dem Drop-on-Powder-Verfahren wird der Schlicker vorzugsweise in ein Bett von $ZrO_2$-Partikeln gedruckt.

Vorzugsweise wird ein Grünkörper aus dem Schlicker hergestellt, indem man den Schlicker schichtweise in einem Inkjet-Druckverfahren zu der geometrischen Form des Grünlings formt. In dem erfindungsgemäßen Verfahren werden vorzugsweise kommerziell verfügbare, hochauflösende industrielle Multi-Düsen-Druckköpfe verwendet. Zum gleichzeitigen Verdrucken mehrerer Schlicker werden vorzugweise Drucker mit mehreren Druckköpfen eingesetzt, die jeweils aus unterschiedlichen Reservoiren gespeist werden, so dass zwei oder mehr unterschiedliche Schlicker verdruckt werden können.

Zur Herstellung des Grünlings kann zusammen mit dem Keramikschlicker ein Stützmaterial verdruckt werden. Stützmaterialien sind Materialien, die beim Druck von Unterschnitten, Überhängen oder Hohlstellen eingesetzt und nach dem Druck wieder aus dem Körper entfernt werden. Das Verdrucken des Stützmaterials erfolgt vorzugweise mit einem separaten Druckkopf. Das Stützmaterial enthält vorzugsweise ausschließlich organische Bestandteile, die beim Entbindern und Sintern vollständig entfernt werden. Als Stützmaterialien eignen sich insbesondere die zur Herstellung der Schlicker verwendeten Bindemittel. Bevorzugte Stützmaterialen sind in folgenden Patenten beschrieben: US 9,138,981 B2, US 8,460,451 B2, US 7,176,253 B2, US 7,399,796 B2 und US 9,534,103 B2. Anders als die im Schlicker vorhandenen organischen Bestandteile lassen sich die Stützmaterialen relativ leicht entfernen, weil sie nicht in das Keramikmaterial eingeschlossen sind.

Der durch schichtweisen Aufbau erhaltene, ungesinterte Körper wird als Grünkörper oder Grünling bezeichnet. Der Grünkörper wird vorzugsweise getrocknet. Das Trocken kann schichtweise und/oder nach der Fertigstellung des Grünkörpers in einem separaten Verfahrensschritt (ii) erfolgen, vorzugsweise mit Hilfe eines Luftstroms und/oder IR-Strahlung. Eine schichtweise Trocknung und insbesondere eine schichtweise Trocknung mit anschließender Trocknung des Grünköpers sind bevorzugt.

Die Trocknung erfolgt vorzugsweise bei einer Temperatur von 10 bis 100°C, vorzugsweise 20 bis 80°C und besonders bevorzugt von 20 bis 60°C.

Weiter bevorzugt erfolgt die Trocknung bei einer relativen Luftfeuchtigkeit 20 bis 90%, vorzugsweise 30 bis 90% und besonders bevorzugt von 40 bis 90%.

Die Dauer der Trocknung beträgt vorzugsweise 0,1 bis 12 h, besonders bevorzugt 0,1 bis 6 h und ganz besonders bevorzugt 1 bis 6 h.

Die Trocknung des gedruckten Bauteils oder der einzelnen gedruckten Schichten kann innerhalb des Druckers oder, im Fall des gedruckten Bauteils, auch nach dem Druckprozess in einem Klimaschrank erfolgen. Die Trocknung kann jeweils mit oder ohne Konvektion, mittels Infrarotstrahlung und/oder Mikrowellen erfolgen. Gemäß einer besonders bevorzugten Ausführungsform des erfindungsgemäßen Verfahrens erfolgt die Trocknung des Grünkörpers als Teil der Sinterung in Schritt (iii).

Der nach der Trocknung erhaltene Grünkörper zeichnet sich durch eine überraschend hohe Dichte aus. Der Grünkörper hat vorzugsweise eine Dichte von 3,3 bis 4,0 $g/cm^3$, besonders bevorzugt von 3,35 bis 3,9 $g/cm^3$ und ganz besonders bevorzugt 3,4 bis 3,9 $g/cm^3$ hat. Die Dichte wird mittels Quecksilberporosimetrie nach ISO 15901-1:2016 bestimmt.

In einer weiteren bevorzugten Ausführungsform hat der Grünkörper ein Porenvolumen von 0,08 bis 0,14 $cm^3/g$, insbesondere 0,08 bis 0,12 $cm^3/g$ und besonders bevorzugt 0,08 bis 0,10 $cm^3/g$. Das Porenvolumen wird mittels Quecksilberporosimetrie nach ISO 15901-1:2016 bestimmt.

In einer anderen bevorzugten Ausführungsform hat der Grünkörper einen Porendurchmesser von 0,02 bis 0,12 $\mu$m, insbesondere 0,03 bis 0,10 $\mu$m und besonders bevorzugt 0,04 bis 0,08 $\mu$m, gemessen als $d_{50}$-Wert bezogen auf das Volumen der Teilchen. Der Porendurchmesser wird mittels Quecksilberporosimetrie nach ISO 15901-1:2016 bestimmt.

Im Anschluss an die Trocknung kann der Grünkörper gegebenenfalls in einem weiteren Schritt durch das Aufbringen einer Färbelösung individuell eingefärbt werden. Vorzugsweise wird die Färbelösung mit Hilfe einer Sprühvorrichtung, bevorzugt einer robotergesteuerten Sprühvorrichtung, aufgebracht. Mit dieser individuellen Farbgebung können örtlich die Transluzenz und/oder die Farbe definiert eingestellt werden. Die Transluzenz wird hierbei durch Lösungen, die bevorzugt Yttrium-, Lanthan-, Gadolinium-, oder Yb-Ionen und deren Mischungen enthalten, eingestellt. Die Farbe wird durch Lösungen, die Fe-, Mn-, Cr-, Co-, Ni-, Ce-, Pr-, Tb-, Bi-, Er- Ionen und deren Mischungen enthalten, eingestellt.

Der Grünkörper kann aufgrund seines geringen Gehalts an organischen Komponenten nach dem Drucken und gegebenenfalls anschließender Trocknung direkt gesintert werden. Eine separate Wärmebehandlung zum Entfernen der organische Bestandteile (Entbinderung) ist nicht erforderlich.

Falls das Trocknen des Grünkörpers im Verlauf der Sinterung erfolgen soll, wird der gedruckte Grünkörper hierzu vorzugsweise auf einem geeigneten Träger in einen Sinterofen mit regelbarem Ofenkopf gestellt. Der Grünkörper wird bei geöffnetem Ofenkopf und Raumtemperatur in den Ofen gestellt, dann die im Ofenkopf befindlichen Heizelemente erwärmt, vorzugsweise auf eine Temperatur von 30 bis 400°C und der Ofenkopf dann langsam abgesenkt. Nachdem der Ofenkopf den Ofen geschlossen hat erfolgt die Sinterung. Eine Trocknung des Grünlings im Verlauf der Sinterung kommt insbesondere dann in Betracht, wenn der Grünling eine Restfeuchte von weniger als 5 Gew.-% aufweist.

Die Grünkörper zeichnen sich dadurch aus, dass sie bei der Dichtsinterung eine nur geringe lineare Schwindung erfahren. Dadurch wird die Erzeugung von dentalen Restaurationen mit genau den gewünschten Dimensionen erleichtert und deren Passgenauigkeit verbessert.

Es ist bevorzugt, dass die Grünkörper eine lineare Schwindung von weniger als 18%, insbesondere weniger als 17% und besonders bevorzugt weniger als 16% haben. Die lineare Schwindung S ergibt sich aus der folgenden Formel und zu ihrer Bestimmung werden Messungen mittels Netzsch-Dilatometer DIL402 Supreme in einem Temperaturbereich von 20°C bis 1550°C bei einer Aufheizgeschwindigkeit von 2 K/min an Prüfkörpern der Dimensionen Länge = 25mm $\pm$ 1mmm, Breite = 5mm $\pm$ 0,5mm und Höhe = 4mm $\pm$ 0,5mm durchgeführt.
Lineare Schwindung:

$$S = \frac{L\ddot{a}nge\ roh - L\ddot{a}nge\ gesintert}{L\ddot{a}nge\ roh} * 100\ in\ \%$$

Aus der linearen Schwindung S kann die Volumenschwindung $S_{Vol}$ gemäß der folgenden Formel berechnet werden:
Volumenschwindung:

$$S_{Vol} = \left[1 - \left(1 - \frac{Lineare\ Schwindung}{100}\right)^3\right] * 100\ in\ \%$$

Die erfindungsgemäßen Grünkörper werden in Schritt (iii) vorzugsweise bei einer Sintertemperatur von 1200 bis 1600°C, insbesondere 1300 bis 1550°C, besonders bevorzugt 1350 bis 1500°C dichtgesintert. Die Dichtsinterung führt zur Bildung eines keramischen Formkörpers, der eine Dichte von insbesondere mehr als 5,9 g/cm$^3$, bevorzugt mehr als 6,00 g/cm$^3$ und besonders bevorzugt mehr als 6,02 g/cm$^3$ hat. Auch aufgrund dieser hohen Dichte verfügt der erhaltene Formkörper über ausgezeichnete mechanische Eigenschaften.

Es ist ein besonderer Vorteil, dass der gesamte Dichtsinterprozess mit dem Aufheizen von Raumtemperatur, dem Halten bei der maximalen Sintertemperatur und dem Abkühlen einen nur sehr kurzen Zeitraum in Anspruch nimmt und nach seinem Abschluss dennoch Formkörper und insbesondere dentale Restaurationen mit der angestrebten hohen Transluzenz und sehr guten mechanischen Eigenschaften erhalten werden. Das erfindungsgemäße Verfahren ist damit konventionellen Verfahren überlegen, die einen sehr langen Zeitraum benötigen, um durch Sinterung eine dentale Restauration mit vergleichbarer Transluzenz zu erzeugen. Das erfindungsgemäße Verfahren vereint damit den Vorteil einer sehr kurzen Verfahrensdauer mit dem der sehr guten optischen und mechanischen Eigenschaften der erzeugten dentalen Restauration.

In einer bevorzugten Ausführungsform des erfindungsgemäßen Verfahrens beträgt der Zeitraum für das Aufheizen des Grünkörpers von Raumtemperatur auf die Sintertemperatur für das Dichtsintern, das Halten bei der Sintertemperatur und das Abkühlen auf die Endtemperatur nicht mehr als 6 Stunden, vorzugsweise nicht mehr als 4 Stunden und besonders bevorzugt nicht mehr als 2 Stunden. Dabei wird unter "Endtemperatur" eine Temperatur verstanden, bei der die Probe in die Hand genommen werden kann, und sie beträgt insbesondere 15 bis 80°C, bevorzugt 25 bis 60°C und besonders bevorzugt etwa 50°C. Unter "Raumtemperatur" wird eine Temperatur von insbesondere 15 bis 30°C, bevorzugt 20 bis 25°C und besonders bevorzugt etwa 25°C verstanden.

Die Aufheizgeschwindigkeit beträgt insbesondere mehr als 10 K/min, bevorzugt mehr als 20 K/min und besonders bevorzugt mehr als 30 K/min. Die Haltezeit beträgt insbesondere weniger als 120 min, bevorzugt weniger als 60 min und besonders bevorzugt weniger als 30 min. Die Abkühlgeschwindigkeit von der Sintertemperatur auf die Endtemperatur beträgt insbesondere mehr als 50 K/min, bevorzugt mehr als 100 K/min und besonders bevorzugt mehr als 150 K/min.

Bevorzugte Bereiche für die Aufheizgeschwindigkeit sind 10 K/min bis 500 K/min, besonders bevorzugt 20 K/min bis 300 K/min und ganz besonders bevorzugt 30 K/min bis 200 K/min.

Die Haltezeiten betragen vorzugsweise 1 Minute bis 60 Minuten, besonders bevorzugt 1 Minute bis 30 Minuten und ganz besonders bevorzugt 1 Minute bis 10 Minuten.

Bevorzugte Bereiche für die Abkühlgeschwindigkeit sind 10 K/min bis 500 K/min, besonders bevorzugt 20 K/min bis 300 K/min und ganz besonders bevorzugt 30 K/min bis 200 K/min.

Durch die geringe lineare Schwindung, die der geformte erfindungsgemäße Grünkörper bei der Dichtsinterung erfährt, wird die Erzeugung von dentalen Restaurationen mit genau den gewünschten Dimensionen erleichtert und deren Passgenauigkeit verbessert. Es versteht sich von selbst, dass die Schwindung beim Sintern beim Druckvorgang entsprechend zu berücksichtigen ist, d.h. die Formkörper werden maßstabsgerecht vergrößert gedruckt, so dass sie nach dem Sintern die gewünschten Dimensionen aufweisen.

In einer bevorzugten Ausführungsform wird

(a) der Grünkörper auf eine Temperatur $T_1$ aufgeheizt,
(b) auf eine Temperatur $T_2$ gegebenenfalls weiter aufgeheizt und bei der Temperatur $T_2$ gehalten und gesintert und
(c) auf eine Temperatur $T_3$ abgekühlt,

wobei die Temperatur $T_1$ 0 bis 500 K, insbesondere 10 bis 250 K, vorzugsweise 25 bis 200 K und besonders bevorzugt 50 bis 100 K unterhalb der Temperatur $T_2$ liegt und Schritt (a) bei niedrigerem Druck als Schritt (b) erfolgt. Dabei ist es weiter bevorzugt, dass der Druck in Schritt (a) weniger als 200 mbar, vorzugsweise weniger als 100 mbar und besonders bevorzugt weniger als 50 mbar beträgt und insbesondere im Bereich von 0,1 bis 200 mbar, vorzugsweise 1 bis 150 mbar und am besonders bevorzugt 50 bis 100 mbar liegt. Ebenfalls ist es bevorzugt, dass Schritt (b) bei einem Druck von mehr als 500 mbar und insbesondere bei Umgebungsdruck erfolgt und insbesondere in einer sauerstoffhaltigen Atmosphäre wie Luft, mit Sauerstoff angereicherter Luft oder Sauerstoff erfolgt. In einer bevorzugten Ausführungsform wird die zum Aufheizen verwendete Heizkammer während Schritt (b) kontinuierlich mit einer sauerstoffhaltigen Atmosphäre, vorzugsweise Luft, mit Sauerstoff angereicherter Luft oder Sauerstoff, durchströmt.

Der nach der Dichtsinterung erhaltene Formkörper kann gegebenenfalls noch mit einer Verblendung versehen, poliert und/oder glasiert werden.

Bei der durch das erfindungsgemäße Verfahren hergestellten dentalen Restauration handelt es sich insbesondere um eine Brücke, ein Inlay, ein Onlay, eine Krone, ein Veneer, ein Implantat, eine Schale oder ein Abutment.

Die Erfindung wird im Folgenden anhand von Ausführungsbeispielen näher erläutert.

**Ausführungsbeispiele**

**Beispiel 1**

_Suspension mit 76 Gew.-% Zirkonoxid_

In 194,4 g destilliertem Wasser wurden nacheinander 3,15 g eines Citronensäure- oder Citronensäuresalzhaltigen Dispergiermittels (Dolapix CE64, Firma Zschimmer & Schwarz) und 1,5 g Tetramethylammoniumhydroxid gelöst. Die Lösung hatte einen pH-Wert von 10-10,5.

Diese Lösung wurde in den Vorratsbehälter einer MicroCer-Rührwerkskugelmühle (Firma Netzsch) gegeben, deren Mahlraum sowie Rotor aus Zirkonoxid bestand. Der Mahlraum wurde mit 60 ml Zirkonoxid-Mahlperlen mit einem Durchmesser von 0,2-0,3 mm (Firma Tosoh) gefüllt. Bei einer Umdrehungsgeschwindigkeit des Rotors von 1500 U/min wurde die Lösung mit einer Schlauchpumpe (Schlauchinnendurchmesser 8 mm) kontinuierlich durch den Mahlraum gepumpt. Zu der Lösung im Vorratsbehälter wurden dann unter Rühren kontinuierlich 630 g Zirkonoxidpulver zugegeben, welches mit 3 Mol.-% $Y_2O_3$ teilstabilisiert war (TZ-PX-245 von TOSOH Corporation, Primärpartikelgröße: 40 nm). Nach vollständiger Zugabe des Zirkonoxidpulvers wurde das erhaltene Gemisch für 45 min kontinuierlich durch den Mahlraum und wieder in den Vorratsbehälter mit einer Rate von ca. 40 1/h gepumpt. Die auf diese Weise hergestellte Suspension wurde in einen Kunststoffmessbecher überführt und mittels eines Magnetrührers sehr langsam gerührt, um eingeschlossene Luftblasen zu entfernen. Es wurde zusätzlich ein Tropfen eines Alkylpolyalkylenglykolethers als Entschäumer (Contraspum, Firma Zschimmer & Schwarz) zugegeben.

Die erhaltene Suspension hatte einen Gehalt an Zirkonoxid von 76 Gew.-%. Die Viskosität $\eta$ der Suspension betrug 7,25 mPas (bei einer Scherrate von 500 s$^{-1}$ und einer Temperatur von 25°C) .

**Beispiel 2**

*Herstellung von Prüfkörpern mittel 3D-Inkjet Drucken*

Für den Druckprozess wurde ein Inkjet Druckkopf (Ricoh MH5421F) mit Fluidzirkulation direkt an den Düsen verwendet. Der Druckkopf wurde mit einem konstanten Überdruck (von 30-120 mBar) an der Versorgungsseite und mit einem konstanten Unterdruck (von -40 bis -150 mBar) an der Rücklaufseite betrieben. Mit dem Schlicker aus Beispiel 1 wurden Prüfkörper mit den folgenden Abmessungen gedruckt: Länge = 25 mm $\pm$ 1 mm, Breite = 5 mm $\pm$ 0,5 mm und Höhe = 4 mm $\pm$ 0,5 mm. Die lineare Schwindung betrug 15,43%. Die Prüfkörper wurden bei 500°C entbindert und hatten dann folgende Eigenschaften: Dichte: 3,678 g/cm$^3$; Länge = 5 mm $\pm$ 1 mm, Breite = 5 mm $\pm$ 1 mm und Höhe = 10 mm $\pm$ 1 mm. Die Prüfkörper wurden anschließend nach dem folgenden Temperaturprogramm im Sinterofen (Programat CS4 von Ivoclar Vivadent AG) dichtgesintert:

|  |  |
|---|---|
| ca. 25°C bis 900°C | (6,7 min) |
| 900°C bis 1460°C | (11,2 min) |
| 1460°C bis 1460°C | (5,0 min) |
| 1460°C bis 1200°C | (3,7 min) |
| 1200°C bis ca. 1000-950°C | (45-50 s) (Ofen öffnet) |
| 950°C-1000°C bis ca. 50°C | (7 min) |

**Beispiel 3**

*Suspension mit 83 Gew.-% Zirkonoxid*

Zur Herstellung einer Suspension mit 83 Gew.-% Zirkonoxid und deren Verarbeitung zu Prüfkörpern (A) und Blöcken für die "chairside"-Anwendung (B) wurde Beispiel 1 mit der Abweichung wiederholt, dass die Lösung 164,5 g destilliertes Wasser, 4,05 g Citronensäure- bzw. Citronensäuresalz-haltiges Dispergiermittel (Dolapix CE64, Firma Zschimmer & Schwarz) und 1,5 g Tetramethylammoniumhydroxid enthielt und 810 g Zirkonoxidpulver zugegeben wurden, welches mit 5 Mol.-% $Y_2O_3$ teilstabilisiert war (TZ-PX-430 von TOSOH Corporation, Primärpartikelgröße: 90 nm) und die Vorsinterung bei 900°C für 2 h durchgeführt wurde, wobei die Aufheizgeschwindigkeit ebenfalls 0,250 K/min betrug.

Die Viskosität $\eta$ der Suspension betrug 7,0 mPas (bei einer Scherrate von 1000 s$^{-1}$ und einer Temperatur von 25°C).

**Beispiel 4**

*Herstellung von Prüfkörpern mittel 3D-Inkjet Drucken*

Auf die in Bespiel 2 beschriebene Weise wurden mit dem Schlicker aus Beispiel 3 Prüfkörper hergestellt. Die lineare Schwindung betrug 14,43%. Die Prüfkörper wurden bei 500°C entbindert und hatten dann folgende Eigenschaften: Dichte: 3,791 g/cm$^3$; Länge = 5mm $\pm$ 1mm, Breite = 5mm $\pm$ 1mm und Höhe = 10mm $\pm$ 1mm). Die Prüfkörper wurden anschließend mit dem in Beispiel 2 beschriebenen Temperaturprogramm im Sinterofen (Programat CS4 von Ivoclar Vivadent AG) dichtgesintert.

**Beispiel 5**

*Suspension mit 80 Gew.-% Zirkonoxid*

Zur Herstellung einer Suspension mit 80 Gew.-% Zirkonoxid und deren Verarbeitung zu Prüfkörpern (A) wurde Beispiel 1 mit der Abweichung wiederholt, dass die Lösung 179,5 g destilliertes Wasser, 3,6 g Citronensäure- bzw. Citronensäuresalz-haltiges Dispergiermittel (Dolapix CE64, Firma Zschimmer & Schwarz) und 1,5 g Tetramethylammoniumhydroxid enthielt und 720 g Zirkonoxidpulver zugegeben wurden, welches mit 4,25 Mol.-% $Y_2O_3$ teilstabilisiert war (TZ-PX-551 von TOSOH Corporation, Primärpartikelgröße: 90 nm).

Die Viskosität η der Suspension betrug 14,6 mPas (bei einer Scherrate von 500 $s^{-1}$ und einer Temperatur von 25°C).

**Beispiel 6**

*Herstellung von Prüfkörpern mittel 3D-Inkjet Drucken*

Auf die in Bespiel 2 beschriebene Weise wurden mit dem Schlicker aus Beispiel 5 Prüfkörper hergestellt. Die lineare Schwindung betrug 14,59%. Die Prüfkörper wurden bei 500°C entbindert und hatten dann eine Dichte: 3,780 $g/cm^3$.

**Beispiel 7**

*Suspension mit 83 Gew.-% Zirkonoxid*

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- bzw. Citronensäuresalz-haltiges Dispergiermittel (Dolapix CE64, Firma Zschimmer & Schwarz) und 1,5 g Tetramethylammoniumhydroxid enthielt und 810 g Zirkonoxidpulver zugegeben wurden, welches mit 3 Mol.-% $Y_2O_3$ teilstabilisiert war (TZ-PX-245 von TOSOH Corporation, Primärpartikelgröße: 40 nm).

**Beispiel 8**

*Herstellung von Prüfkörpern mittel 3D-Inkjet Drucken*

Auf die in Bespiel 2 beschriebene Weise wurden mit dem Schlicker aus Beispiel 7 Prüfkörper hergestellt. Die Prüfkörper wurden bei 500°C entbindert und hatten dann folgende Eigenschaften:

- Porenvolumen: 0,1139 $cm^3/g$
- Porenradius: 0,0190 μm
- Dichte: 3,562 $g/cm^3$

**Beispiel 9**

*Suspension mit 83 Gew.-% Zirkonoxid*

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- bzw. Citronensäuresalz-haltiges Dispergiermittel (Dolapix CE64, Firma Zschimmer & Schwarz) und 2,0 g Tetramethylammoniumhydroxid enthielt und 810 g Zirkonoxidpulver zugegeben wurden, welches mit 4,25 Mol-% $Y_2O_3$ teilstabilisiert war (TZ-PX-551 von TOSOH Corporation, Primärpartikelgröße: 90 nm).

**Beispiel 10**

*Herstellung von Prüfkörpern mittel 3D-Inkjet Drucken*

Auf die in Bespiel 2 beschriebene Weise wurden mit dem Schlicker aus Beispiel 9 Prüfkörper hergestellt. Die Prüfkörper wurden bei 500°C entbindert und hatten dann folgende Eigenschaften:

- Porenvolumen: 0,1109 $cm^3/g$
- Porenradius: 0,0248 μm
- Dichte: 3,547 $g/cm^3$

**Beispiel 11**

*Suspension mit 83 Gew.-% Zirkonoxid*

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- bzw. Citronensäuresalz-haltiges Dispergiermittel (Dolapix CE64, Firma Zschimmer & Schwarz) und 2,0 g Tetramethylammoniumhydroxid enthielt und 810 g Zirkonoxidpulver zugegeben wurden, welches mit 5,0 Mol-% $Y_2O_3$ teilstabilisiert war (TZ-PX-430 von TOSOH Corporation, Primärpartikelgröße: 90 nm).

**Beispiel 12**

*Herstellung von Prüfkörpern mittel 3D-Inkjet Drucken*

Auf die in Bespiel 2 beschriebene Weise wurden mit dem Schlicker aus Beispiel 11 Prüfkörper hergestellt. Die Prüfkörper wurden bei 500°C entbindert und hatten dann folgende Eigenschaften:

- Porenvolumen: 0,1056 $cm^3$/g
- Porenradius: 0,0253 $\mu$m
- Dichte: 3,783 g/$cm^3$

**Patentansprüche**

1. Verwendung eines Schlickers, der Zirkonoxid enthält, das in einem flüssigen Medium suspendiert ist, wobei der Schlicker einen Gehalt an Zirkonoxid von 68 bis 88 Gew.-%, bevorzugt 70 bis 86 Gew.-% und besonders bevorzugt 75 bis 85 Gew.-% aufweist, und nicht mehr als 3 Gew.-%, bevorzugt nicht mehr als 2 Gew.-% und besonders bevorzugt nicht mehr als 1 Gew.-%, bezogen auf die Gesamtmasse des Schlickers an organischen Komponenten enthält, wobei das Zirkonoxid in dem Schlicker eine Teilchengröße von 50 bis 250 nm gemessen als $d_{50}$-Wert und bezogen auf das Volumen der Teilchen aufweist, und wobei der Schlicker eine Viskosität von 5 bis 1000 mPas, gemessen bei einer Scherrate von 0,1 bis 1000 $s^{-1}$ und einer Temperatur von 25°C, hat, zur Herstellung von Keramikformteilen mit einem Inkjet-Druckverfahren.

2. Verwendung nach Anspruch 1, wobei das Zirkonoxid in dem Schlicker eine Teilchengröße von 60 bis 250 nm und bevorzugt 80 bis 250 nm, gemessen als $d_{50}$-Wert und bezogen auf das Volumen der Teilchen, aufweist.

3. Verwendung nach einem der Ansprüche 1 bis 2, wobei das Zirkonoxid mit $Y_2O_3$, $La_2O_3$, $CeO_2$, MgO und/oder CaO stabilisiert ist und insbesondere mit 2 bis 14 Mol-%, bevorzugt 2 bis 10 Mol-% und besonders bevorzugt 2 bis 8 Mol-% dieser Oxide, bezogen auf den Gehalt an Zirkonoxid, stabilisiert ist.

4. Verwendung nach einem der Ansprüche 1 bis 3, wobei das flüssige Medium Wasser enthält.

5. Verwendung nach einem der Ansprüche 1 bis 4, wobei der Schlicker organische Komponenten in einer Menge von 0,05 bis 3 Gew.-%, insbesondere 0,1 bis 3 Gew.-%, bevorzugt 0,1 bis 2 Gew.-% und besonders bevorzugt 0,1 bis 1 Gew.-%, bezogen auf die Gesamtmasse des Schlickers enthält.

6. Verwendung nach einem der Ansprüche 1 bis 5, wobei das flüssige Medium mindestens eine Verbindung ausgewählt aus Aminoalkohol, Glykol, Carbonsäure und Carbonsäuresalz und bevorzugt mindestens eine Verbindung ausgewählt aus Ethanolamin, Ethylenglykol, Dipropylenglykol, Citronensäure und Citronensäuresalz enthält.

7. Verwendung nach einem der Ansprüche 1 bis 6, wobei der Schlicker eine Viskosität von 5 bis 500 mPas und bevorzugt 5 bis 250 mPas, gemessen bei einer Scherrate von 0,1 bis 1000 $s^{-1}$ und einer Temperatur von 25°C hat.

8. Verwendung nach einem der Ansprüche 1 bis 7, wobei der Schlicker ein Gemisch von Zirkonoxidpulvern mit unterschiedlicher Zusammensetzung enthält.

9. Verwendung nach einem der Ansprüche 1 bis 8, wobei das Keramikformteil eine Dentalrestauration ist, vorzugsweise ein Inlay, Onlay, Veneer, eine Krone, Brücke, ein Gerüst, ein Implantat, eine Schale oder ein Abutment.

10. Verfahren zur Herstellung von keramischen Bauteilen, bei dem man

(i) einen Schlicker mit der in einem der Ansprüche 1 bis 8 definierten Zusammensetzung schichtweise zur geometrischen Form des gewünschten Bauteils formt, wobei man vorzugsweise die einzelnen Schichten nach dem Drucken jeweils trocknet,
(ii) man den so erhaltenen Grünling ggf. anschließend trocknet und
(iii) man dann den Grünling sintert,

wobei der schichtweise Aufbau des Grünlings in Schritt (i) durch ein schichtweises Inkjet-Druckverfahren erfolgt.

11. Verfahren nach Anspruch 10, bei dem der in Schritt (i) gebildete Grünkörper in Schritt (ii) bei einer Temperatur von 10 bis 100°C, bevorzugt 20 bis 80°C und besonders bevorzugt 20 bis 60°C getrocknet wird.

12. Verfahren nach Anspruch 10 oder 11, bei dem der Grünkörper eine Dichte von 3,3 bis 4,0 g/cm$^3$, insbesondere 3,35 bis 3,9 g/cm$^3$ und bevorzugt 3,4 bis 3,9 g/cm$^3$
und/oder
ein Porenvolumen von 0,08 bis 0,14 cm$^3$/g, insbesondere 0,08 bis 0,12 cm$^3$/g und bevorzugt 0,08 bis 0,10 cm$^3$/g
und/oder
einen Porendurchmesser von 0,02 bis 0,12 $\mu$m, insbesondere 0,03 bis 0,10 $\mu$m und bevorzugt 0,04 bis 0,08 $\mu$m, gemessen als $d_{50}$-Wert bezogen auf das Volumen der Teilchen, hat.

13. Verfahren nach einem der Ansprüche 10 bis 12 bei dem der Grünkörper bei einer Sintertemperatur von 1200 bis 1600°C, insbesondere 1300 bis 1550°C und bevorzugt 1350 bis 1500°C dichtgesintert wird.

14. Verfahren nach Anspruch 13, bei dem der Zeitraum für das Aufheizen des Grünkörpers von Raumtemperatur auf die Sintertemperatur, das Halten bei der Sintertemperatur und das Abkühlen auf die Endtemperatur nicht mehr als 6 Stunden, bevorzugt nicht mehr als 4 Stunden und besonders bevorzugt nicht mehr als 2 Stunden beträgt.

15. Verfahren nach Anspruch 13 oder 14, bei dem

(a) der Grünkörper auf eine Temperatur $T_1$ aufgeheizt wird,
(b) auf eine Temperatur $T_2$ gegebenenfalls weiter aufgeheizt und bei der Temperatur $T_2$ gehalten und gesintert wird und
(c) auf eine Temperatur $T_3$ abgekühlt wird,

wobei die Temperatur $T_1$ 0 bis 500 K, insbesondere 10 bis 250 K, vorzugsweise 25 bis 200 K und besonders bevorzugt 50 bis 100 K unterhalb der Temperatur $T_2$ liegt und Schritt (a) bei niedrigerem Druck als Schritt (b) erfolgt.

16. Verfahren nach einem der Ansprüche 10 bis 15, bei dem das keramische Bauteil eine dentale Restauration, vorzugsweise ein Inlay, Onlay, Veneer, eine Krone, Brücke, ein Gerüst, ein Implantat, eine Schale oder ein Abutment, ist.

**Claims**

1. Use of a slip that comprises zirconium oxide suspended in a liquid medium for the production of ceramic shaped parts by an inkjet printing process, wherein the slip has a zirconium oxide content of 68 to 88 wt %, preferably 70 to 86 wt % and particularly preferably 75 to 85 wt %, and comprises not more than 3 wt %, preferably not more than 2 wt % and particularly preferably not more than 1 wt % of organic components, relative to the total mass of the slip, wherein the zirconium oxide in the slip exhibits a particle size of 50 to 250 nm, measured as the dso value and relative to the volume of the particles, and wherein the slip has a viscosity of 5 to 1000 mPas, measured at a shear rate of from 0.1 to 1000 s$^{-1}$ and at a temperature of 25 °C.

2. Use according to claim 1, wherein the zirconium oxide in the slip has a particle size from 60 to 250 nm and preferably 80 to 250 nm, measured as the dso value and relative to the volume of the particles.

3. Use according to claim 1 or 2, wherein the zirconium oxide is stabilised with $Y_2O_3$, $La_2O_3$, $CeO_2$, MgO and/or CaO, and in particular is stabilised with 2 to 14 mol %, preferably 2 to 10 mol % and particularly preferably 2 to 8 mol % of these oxides, relative to the zirconium oxide content.

4. Use according to one of claims 1 to 3, wherein the liquid medium comprises water.

5. Use according to one of claims 1 to 4, wherein the slip comprises organic components in an amount of from 0.05 to 3 wt %, in particular 0.1 to 3 wt %, preferably 0.1 to 2 wt % and particularly preferably 0.1 to 1 wt %, relative to the total mass of the slip.

6. Use according to one of claims 1 to 5, wherein the liquid medium comprises at least one compound selected from amino alcohol, glycol, carboxylic acid and carboxylic acid salt and preferably at least one compound selected from ethanolamine, ethylene glycol, dipropylene glycol, citric acid and citric acid salt.

7. Use according to one of claims 1 to 6, wherein the slip has a viscosity of from 5 to 500 mPas, and preferably 5 to 250 mPas, measured at a shear rate of from 0.1 to 1000 $s^{-1}$ and at a temperature of 25 °C.

8. Use according to one of claims 1 to 7, wherein the slip comprises a mixture of zirconium oxide powders with different compositions.

9. Use according to one of claims 1 to 8, wherein the ceramic shaped part is a dental restoration, preferably an inlay, onlay, veneer, a crown, bridge, a framework, an implant, a shell or an abutment.

10. Process for the production of ceramic components, in which

    (i) a slip with the composition defined in one of claims 1 to 8 is shaped in layers into the geometric shape of the desired component, wherein the individual layers are each preferably dried after printing,
    (ii) the thus-obtained green body is then optionally dried and
    (iii) the green body is then sintered,

    wherein the layered construction of the green body in step (i) is effected by a layered inkjet printing process.

11. Process according to claim 10, in which the green body formed in step (i) is dried in step (ii) at a temperature from 10 to 100 °C, preferably 20 to 80 °C and particularly preferably 20 to 60 °C.

12. Process according to claim 10 or 11, in which the green body has a density of from 3.3 to 4.0 $g/cm^3$, in particular 3.35 to 3.9 $g/cm^3$ and preferably 3.4 to 3.9 $g/cm^3$
    and/or
    a pore volume of from 0.08 to 0.14 $cm^3/g$, in particular 0.08 to 0.12 $cm^3/g$ and preferably 0.08 to 0.10 $cm^3/g$
    and/or
    a pore diameter of from 0.02 to 0.12 $\mu$m, in particular 0.03 to 0.10 $\mu$m and preferably 0.04 to 0.08 $\mu$m, measured as the dso value relative to the volume of the particles.

13. Process according to one of claims 10 to 12, in which the green body is densely sintered at a sintering temperature from 1200 to 1600 °C, in particular 1300 to 1550 °C, and preferably 1350 to 1500 °C.

14. Process according to claim 13, in which the period for heating up the green body from room temperature to the sintering temperature, maintaining the sintering temperature and cooling down to the final temperature is not more than 6 hours, preferably not more than 4 hours and particularly preferably not more than 2 hours.

15. Process according to claim 13 or 14, in which

    (a) the green body is heated up to a temperature $T_1$,
    (b) is optionally further heated up to a temperature $T_2$ and held and sintered at the temperature $T_2$ and
    (c) is cooled down to a temperature $T_3$,

    wherein the temperature $T_1$ is 0 to 500 K, in particular 10 to 250 K, preferably 25 to 200 K and particularly preferably 50 to 100 K below the temperature $T_2$ and step (a) is effected at a lower pressure than step (b).

16. Process according to one of claims 10 to 15, in which the ceramic component is a dental restoration, preferably an inlay, onlay, veneer, a crown, bridge, a framework, an implant, a shell or an abutment.

**Revendications**

1.  Utilisation d'une barbotine qui contient de l'oxyde de zirconium en suspension dans un milieu liquide, laquelle barbotine présente une teneur en oxyde de zirconium de 68 à 88 % en poids, de préférence de 70 à 86 % en poids et mieux encore de 75 à 85 % en poids, et ne contient pas plus de 3 % en poids, de préférence pas plus de 2 % en poids et mieux encore pas plus de 1 % en poids, par rapport au poids total de la barbotine, de composants organiques, étant entendu que l'oxyde de zirconium contenu dans la barbotine présente une taille de particules, exprimée par la valeur de $d_{50}$ rapportée au volume des particules, de 50 à 250 nm, et laquelle barbotine présente une viscosité, mesurée avec une vitesse de cisaillement de 0,1 à 1000 $s^{-1}$ et à la température de 25 °C, de 5 à 1000 mPa.s, pour la fabrication de pièces façonnées en céramique, selon un procédé d'impression par jet d'encre.

2.  Utilisation conforme à la revendication 1, dans laquelle l'oxyde de zirconium contenu dans la barbotine présente une taille de particules, exprimée par la valeur de $d_{50}$ rapportée au volume des particules, de 60 à 250 nm, et de préférence de 80 à 250 nm.

3.  Utilisation conforme à l'une des revendications 1 et 2, dans laquelle l'oxyde de zirconium est stabilisé à l'aide de $Y_2O_3$, de $La_2O_3$, de $CeO_2$, de MgO et/ou de CaO, et en particulier, est stabilisé avec de 2 à 14 % en moles, de préférence de 2 à 10 % en moles et mieux encore de 2 à 8 % en moles de ces oxydes, par rapport à la quantité d'oxyde de zirconium.

4.  Utilisation conforme à l'une des revendications 1 à 3, dans laquelle le milieu liquide contient de l'eau.

5.  Utilisation conforme à l'une des revendications 1 à 4, dans laquelle la barbotine contient des composants organiques en une proportion de 0,05 à 3 % en poids, en particulier de 0,1 à 3 % en poids, de préférence de 0,1 à 2 % en poids et mieux encore de 0,1 à 1 % en poids, par rapport au poids total de la barbotine.

6.  Utilisation conforme à l'une des revendications 1 à 5, dans laquelle le milieu liquide contient au moins un composé choisi parmi un amino-alcool, un glycol, un acide carboxylique et un sel d'acide carboxylique, et de préférence, au moins un composé choisi parmi de l'éthanolamine, de l'éthylène-glycol, du dipropylène-glycol, de l'acide citrique et un sel d'acide citrique.

7.  Utilisation conforme à l'une des revendications 1 à 6, dans laquelle la barbotine présente une viscosité, mesurée avec une vitesse de cisaillement de 0,1 à 1000 $s^{-1}$ et à la température de 25 °C, de 5 à 500 mPa.s et de préférence de 5 à 250 mPa.s.

8.  Utilisation conforme à l'une des revendications 1 à 7, dans laquelle la barbotine contient un mélange de poudres d'oxyde de zirconium de différentes compositions.

9.  Utilisation conforme à l'une des revendications 1 à 8, dans laquelle la pièce façonnée en céramique est une pièce de restauration dentaire, et de préférence une incrustation inlay ou onlay, une facette veneer, une couronne, un bridge, une armature, un implant, une coquille ou un pilier.

10. Procédé de fabrication de pièces élaborées en céramique, dans lequel

    i) on façonne une barbotine, qui présente une composition telle que définie dans l'une des revendications 1 à 8, en la disposant couche après couche jusqu'à obtenir la forme géométrique de la pièce voulue, étant entendu que, de préférence, on fait sécher chacune des couches individuelles après l'impression,
    ii) le cas échéant, on fait finalement sécher l'ébauche crue ainsi obtenue,
    iii) et l'on fritte ensuite cette ébauche crue,

    étant entendu que dans l'étape (i), on réalise la construction couche après couche de l'ébauche crue selon une procédé d'impression par jet d'encre en couches.

11. Procédé conforme à la revendication 10, dans lequel, dans l'étape (ii), on fait sécher le corps cru construit dans l'étape (i) à une température de 10 à 100 °C, de préférence de 20 à 80 °C, et mieux encore de 20 à 60 °C.

12. Procédé conforme à la revendication 10 ou 11, dans lequel le corps cru présente

- une masse volumique de 3,3 à 4,0 g/cm$^3$, en particulier de 3,35 à 3,9 g/cm$^3$ et de préférence de 3,4 à 3,9 g/cm$^3$,
- et/ou un volume de pores de 0,08 à 0,14 cm$^3$/g, en particulier de 0,08 à 0,12 cm$^3$/g et de préférence de 0,08 à 0,10 cm$^3$/g,
- et/ou un diamètre de pores, exprimé par la valeur de $d_{50}$ rapportée au volume des particules, de 0,02 à 0,12 $\mu$m, en particulier de 0,03 à 0,10 $\mu$m et de préférence de 0,04 à 0,08 $\mu$m.

13. Procédé conforme à l'une des revendications 10 à 12, dans lequel le corps cru est fritté-densifié à une température de frittage de 1200 à 1600 °C, en particulier de 1300 à 1550 °C et de préférence de 1350 à 1500 °C.

14. Procédé conforme à la revendication 13, dans lequel le laps de temps que durent le chauffage du corps cru depuis la température ambiante jusqu'à la température de frittage, son maintien à la température de frittage et son refroidissement jusqu'à la température finale ne vaut pas plus de 6 heures, de préférence pas plus de 4 heures, et mieux encore pas plus de 2 heures.

15. Procédé conforme à la revendication 13 ou 14, dans lequel le corps cru

   a) est chauffé jusqu'à une température $T_1$,
   b) le cas échéant, est chauffé davantage, jusqu'à une température $T_2$, puis maintenu et fritté à cette température $T_2$,
   c) et refroidi jusqu'à une température $T_3$,

   étant entendu que la température $T_1$ est inférieure de 0 à 500 K, en particulier de 10 à 250 K, de préférence de 25 à 200 K et mieux encore de 50 à 500 K à la température $T_2$, et que l'étape (a) est effectuée sous une plus basse pression que l'étape (b).

16. Procédé conforme à l'une des revendications 10 à 15, dans lequel la pièce élaborée en céramique est une pièce de restauration dentaire, et de préférence une incrustation inlay ou onlay, une facette veneer, une couronne, un bridge, une armature, un implant, une coquille ou un pilier.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2233449 A1
- EP 2529694 A1
- WO 2015056230 A1
- US 9138981 B2
- US 8460451 B2
- US 7176253 B2
- US 7399796 B2
- US 9534103 B2

**In der Beschreibung aufgeführte Nicht-Patentliteratur**

- **A. GEBHARDT.** *Vision Rapid Prototyping,* 2006, vol. 83, 7-12